# FASCICULE DE BREVET EUROPEEN

(11) **EP 1 269 924 B1**
(45) Date de publication et mention de la délivrance du brevet: **13.12.2006**
(21) Numéro de dépôt: 02291573.0
(22) Date de dépôt: 25.06.2002
(51) Int. Cl.: A61B 17/15

(54) **Dispositif de positionnement d'un plan de coupe d'un guide de coupe d'un os**
Vorrichtung zur Positionierung der Schnittebene einer Knochensägenführung
Device for positioning the cutting plane of a bone cutting guide

(30) Priorité: 25.06.2001 FR 0108318
(43) Date de publication de la demande: 02.01.2003
(73) Titulaire: Aesculap Société par actions simplifiée, 52000 Chaumont (FR); Aesculap AG & Co. KG, 78532 Tuttlingen (DE)
(72) Inventeur: Millard, Thierry, 33610 Cestas (FR); Friedrich, Dirk, 78532 Tuttlingen (DE)
(74) Mandataire: Eidelsberg, Olivier Nathan

(56) Documents cités:
- DE-U- 29 704 393
- FR-A- 2 718 010
- FR-A- 2 776 176
- US-A- 4 457 307
- US-A- 6 033 410

## Description

La présente invention concerne un dispositif de positionnement d'un guide de coupe, en vue de couper ou réséquer un os, notamment un tibia et/ou un fémur pour y poser une prothèse du genou.

On connaît dans l'art antérieur, et notamment de la demande de brevet français N° 9803421 (FR 2776 176 AFSA29) du 20 Mars 1998 au nom de la demanderesse, un système de positionnement de guide de coupe de ce genre. Le dispositif de positionnement décrit dans ce document de l'art antérieur présente plusieurs inconvénients. En premier lieu, dans le cas de la résection du tibia, il est particulièrement encombrant, comportant une tige principale et des moyens de fixation au bas du tibia. En second lieu, ce dispositif de positionnement n'est pas universel. C'est-à-dire, un même dispositif de positionnement de guide de coupe ne peut pas convenir à la fois pour un guide de coupe de tibia et pour un guide de coupe de fémur. Comme on peut le voir dans le document de l'art antérieur mentionné précédemment, le dispositif de positionnement de guide de coupe pour le tibia est très différent de son homologue pour le fémur. Par conséquent, les fabricants de dispositifs de ce genre doivent pouvoir être à même de disposer de deux lignes de fabrication pour ces deux types de dispositifs de positionnement et les chirurgiens doivent avoir à leur disposition les deux types de dispositifs de positionnement, ce qui est bien évidemment un inconvénient.

Du document FR-2.776.176, il est décrit un système comportant des premiers moyens de réglage de l'orientation du plan de coupe par rapport à un deuxième axe perpendiculaire l'axe d'ancrage, mais qui ne comporte pas de deuxième moyens de réglage pour régler la hauteur du guide de coupe par rapport au dispositif de positionnement le long du troisième axe perpendiculaire, cette hauteur y étant constante puisque les montants 83 ne sont pas coulissants. En outre, ce système ne peut être utilisé que sur un fémur et ne peut pas être adapté à un tibia.

La présente invention vise un dispositif de positionnement de guide de coupe qui surmonte les inconvénients mentionnés ci-dessus de l'art antérieur et notamment qui est universel, c'est-à-dire qui peut être utilisé aussi bien pour positionner un guide de coupe de tibia qu'un guide de coupe de fémur, et qui est beaucoup moins encombrant que les dispositifs connus de l'art antérieur. En outre, le dispositif suivant l'invention est particulièrement bien adapté à la technique GMCAO (Geste Médico-Chirurgical Associé par Ordinateur).

Il est connu de US-A4457307 un dispositif ayant les caractéristiques du préambule de la revendication 1.

Suivant l'invention, le dispositif de positionnement est tel que défini la revendication 1.

Des perfectionnements font l'objet des revendications 2 à 8.

Suivant l'invention, qui est particulièrement simple de fabrication et de faible encombrement, les premiers moyens d'ancrage sont constitués d'un alésage formé dans le corps du dispositif de positionnement et d'une vis ou broche ou destinée à être vissée ou introduite dans le premier alésage pour être ancrée dans l'os et les deuxièmes moyens d'ancrage sont constitués d'un second alésage parallèle au premier alésage et d'une deuxième vis.
ou broche ou analogue destinée à passer ou à se visser dans le deuxième alésage pour pouvoir être ancrée dans l'os. Les premiers moyens de réglage sont constitués de deux montants montés sensiblement perpendiculairement à la direction des axes d'ancrage, ces deux montants étant reliés aux moyens de fixation du guide de coupe d'une part, et étant reliés l'un à l'autre par un montant transversal, notamment sensiblement perpendiculaire aux deux montants et également perpendiculaire à la direction des axes d'ancrage, et une vis, notamment commandée par une molette, agissant sur le montant transversal pour le déplacer par rapport au corps du dispositif de positionnement dans la direction des axes d'ancrage.

Ce système de réglage est assez simple car le réglage s'effectue par le biais d'une simple translation du montant transversal. Cependant, comme cette translation est effectuée par rapport au corps fixe qui a plusieurs points d'appui sur l'os, à savoir un point d'appui fixe par rapport à l'os, à savoir les points d'ancrage, et un autre point, à savoir un niveau du contact du guide de coupe avec l'os, cette translation se transforme en une rotation du plan de coupe dans un petit domaine de rotation, ce qui permet de bien régler le plan de coupe A/P (antéro-postérieur), c'est-à-dire le plan de coupe dans le plan sagittal par exemple. On a ainsi obtenu un système très simple pour régler cette pente A/P (antéro-postérieure) du genou contrairement au cas des dispositifs de l'art antérieur qui étaient particulièrement compliqués.

Suivant un mode de réalisation préféré de l'invention, les deux montants verticaux coulissent par rapport au corps principal dans la troisième direction sous l'action d'une molette, ce qui permet de régler la hauteur du guide de coupe auquel ils sont fixés par l'intermédiaire des moyens de fixation par rapport au corps du guide de coupe et donc par rapport à l'os auquel le corps de guide de coupe est ancré.

Suivant encore un autre mode de réalisation préféré de l'invention, il est prévu des moyens de réglage fins de l'angle de rotation par rapport au premier axe d'ancrage.

Suivant un mode de réalisation préféré de l'invention, ces moyens de réglage fins sont constitués par le fait que le deuxième alésage est monté mobile sous l'action d'une tige actionnée par molette par rapport au corps principal du dispositif.

Ainsi, une fois que les première et deuxième vis d'ancrage ont été ancrées dans l'os pour définir ce que l'on appelle l'angle de varus/valgus avec une certaine précision, on peut affiner ce positionnement en agissant sur la molette en déplaçant très légèrement en translation par rapport au corps du dispositif de positionnement le deuxième axe d'ancrage.

En jouant ainsi sur les jeux des ancrages du dispositif de positionnement, on peut légèrement modifier l'angle de varus/valgus, notamment dans un domaine de plus ou moins 3°, sans que cela ne détériore l'os de manière grave. Ici encore, une simple translation suffit pour commander un positionnement en rotation. Il s'agit donc d'une structure particulièrement simple du dispositif.

Suivant un mode de réalisation préféré de l'invention, le guide de coupe ou le dispositif de positionnement du guide de coupe comporte un capteur permettant de positionner dans l'espace le plan de coupe, notamment dans un système dit GMCAO, par exemple tel que défini dans le modèle d'utilité allemand au nom de la Société AESCULAP AG N°29704393.5 du 11 Mars 1997.

Suivant un mode de réalisation préféré de l'invention, le dispositif comporte des moyens de fixation, notamment amovibles, au guide de coupe, constitués notamment d'ergots d'encliquetage s'encliquetant dans des logements respectifs dans les guides de coupe.

La présente invention vise également un dispositif de utilisable aussi bien pour un tibia que pour un fémur.

La présente invention peut être utilisée dans un procédé de coupe d'un os, notamment d'un tibia et/ou d'un fémur, dans lequel il est prévu des premiers moyens d'ancrage destinés à ancrer le dispositif de positionnement à l'os de sorte que l'ensemble du dispositif et des moyens de fixation du guide de coupe puisse tourner par rapport à l'axe d'ancrage ; et
des deuxièmes moyens d'ancrage destinés à ancrer le dispositif de positionnement en un deuxième point d'ancrage sur l'os, différent du premier point d'ancrage, de manière à bloquer en une position souhaitée de rotation du dispositif par rapport au premier axe d'ancrage, qui comprend les étapes qui consistent à :
- ancrer d'abord en un point le dispositif de positionnement de guide de coupe suivant un axe d'ancrage,
- une fois celui-ci ancré, le faire pivoter par rapport à l'axe d'ancrage dans une position donnée, et ensuite seulement
- ancrer le dispositif de positionnement en un deuxième point suivant un deuxième axe d'ancrage.

On décrit maintenant plusieurs modes de réalisation de l'invention, donnés uniquement à titre d'illustration, en se reportant aux dessins, dans lesquels :
La figure 1 est une vue en perspective d'un premier mode de réalisation d'un dispositif suivant l'invention ;
La figure 2 est une vue en perspective suivant un autre angle du même dispositif de positionnement, avec à distance un capteur de fémur droit destiné à être fixé à ce dispositif de positionnement et positionné par rapport au fémur également représenté à la figure en vue de résecter le fémur représenté pour y poser une prothèse ;
La figure 3 est une vue identique à celle de la figure 1 d'un autre mode de réalisation de l'invention ; et
La figure 4 est une vue identique à celle de la figure 2 concernant le deuxième mode de réalisation représenté aux figures.

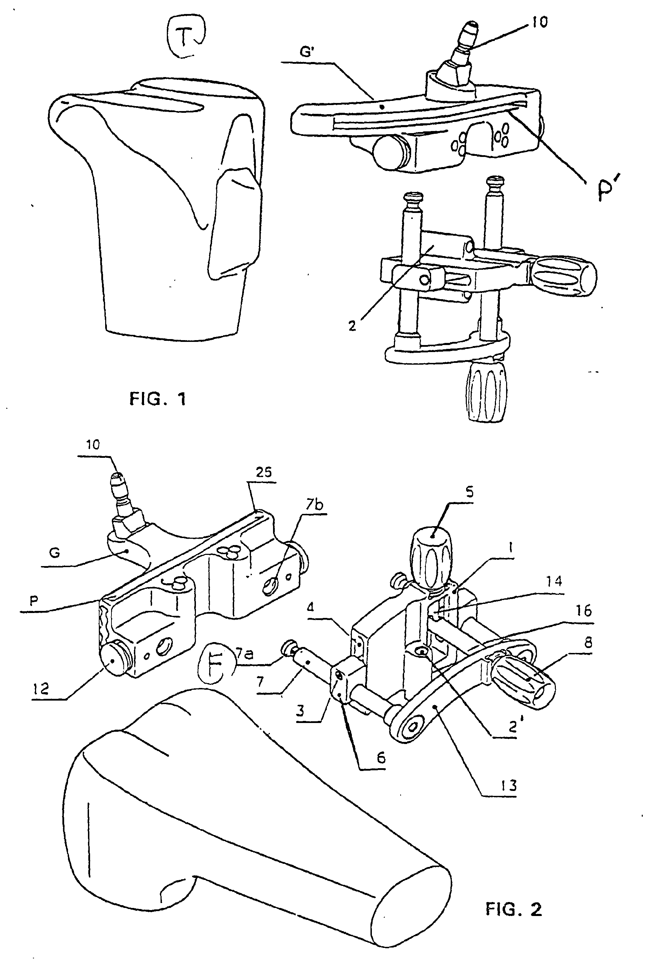

A la figure 2, le dispositif (P) de positionnement d'un guide de coupe (G) comporte un corps 1 de base en forme sensiblement de plaque évidée. L'évidement 4 formé dans la plaque de base s'étend sur sensiblement toute la largeur de la plaque. Deux protubérances font saillies de la plaque 1. Il est formé dans chacune de ces protubérances un alésage 2, 2' longitudinal, définissant chacun un axe d'ancrage respectif parallèle à une première direction, auquel on se référera ci-après par direction de varus/valgus ou direction des axes d'ancrage.

Une tige 3 transversale, logée dans l'évidement 4, traverse de part et d'autre le corps principal 1. Aux deux extrémités de la tige 3 transversale se trouvent deux butées 6 qui viennent buter contre les bords extérieurs de deux ouvertures latérales opposées de l'évidement 4 pour ainsi permettre uniquement le déplacement de la tige 3 en translation à l'intérieur de l'évidement 4, parallèlement à la direction dite de varus/valgus. Deux montants verticaux 7 sont liés fonctionnellement à la tige ou montant transversal 3 par l'intermédiaire des deux butées 6, les deux montants 7 verticaux pouvant coulisser dans deux alésages formés dans les deux butées 6 dans une direction (que l'on appellera ci-après la direction en hauteur) à la fois perpendiculaire à l'axe de la tige transversale et perpendiculaire à la direction dite de varus/valgus. Chacun des montants 7 verticaux comprend à son extrémité supérieure ou libre un ergot 7a respectif destiné à s'encliqueter dans des logements 7b respectifs formés dans le guide de coupe (G), encliquetage actionné par l'intermédiaire de boutons 12 "pistons". Une tige 14 est liée fonctionnellement à la tige 3 transversale. La tige 14 est en partie filetée à son extrémité opposée à la tige 3 de manière à pouvoir être déplacée (vissée ou dévissée) dans la direction des axes d'ancrage par l'intermédiaire d'une molette 5. Par l'action de la molette 5, par l'intermédiaire de la tige 14 qui est liée fonctionnellement à la tige 3 transversale, la tige 3 transversale est commandée dans son déplacement en translation le long de l'axe d'ancrage.

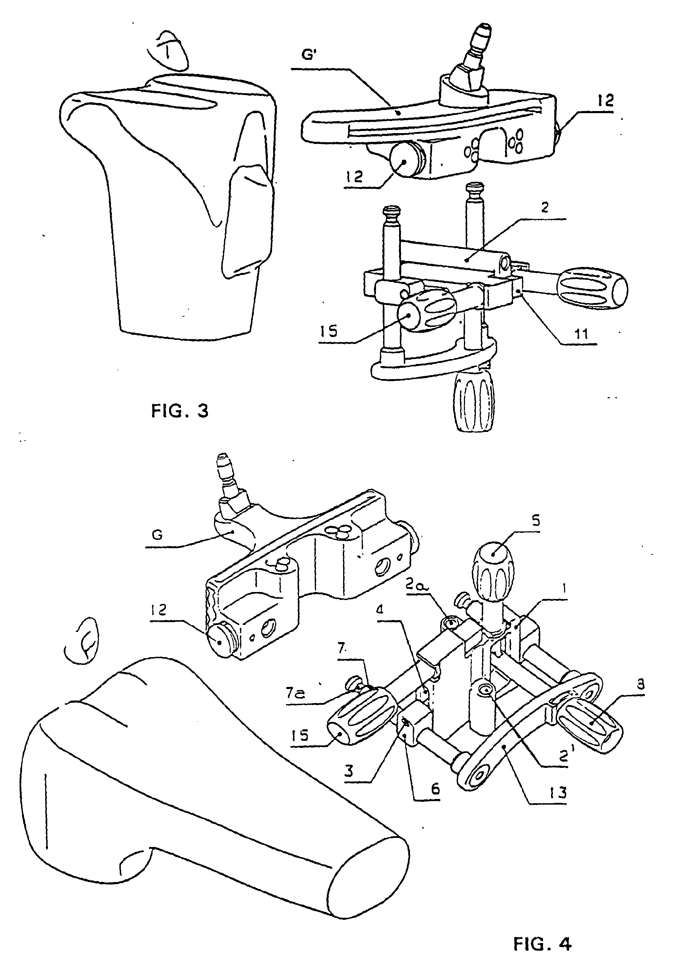

Les deux extrémités inférieures des deux montants 7 verticaux sont reliées entre elles par une base 13. Une tige 16, parallèle aux montants 7 et en partie filetée à une extrémité, passe à travers la base 13, dans un trou taraudé pour ainsi pouvoir être déplacée en y étant vissée ou dévissée par l'intermédiaire d'une molette 8, de sorte que l'on peut régler la distance entre le corps 1 principal et la base 13 et par conséquent la mesure de laquelle les montants 7 verticaux font saillies extérieurement du corps 1 principal. Le guide (G) de coupe représenté à la figure 2 est un guide de coupe pour un fémur (que l'on voit également à la figure 4). Il s'agit d'un guide de coupe classique. Il comporte un capteur 10 servant dans un système dit GMCAO (Geste Médico-Chirurgical Associé par Ordinateur). Une fente 25 définit le plan (P) de coupe suivant lequel l'instrument de coupe (une lame) va être guidé dans la fente. Le procédé de positionnement du guide de coupe est le suivant :

Dans un premier temps, le chirurgien ancre par l'intermédiaire du premier alésage 2 d'ancrage le dispositif sur une face latérale frontale du tibia ou du fémur. Le chirurgien essaye à ce moment-là de placer le dispositif le plus près possible de l'endroit où il souhaite que soit disposé finalement le guide de coupe. Une fois la première vis d'ancrage vissée ou ancrée dans le tibia ou le fémur, le chirurgien peut faire tourner le dispositif de positionnement par rapport à l'axe d'ancrage pour obtenir le positionnement exact qu'il souhaite donné au guide de coupe : c'est-à-dire il règle l'angle de varus/valgus. On dispose ainsi le plan (P') de coupe dans un premier plan aussi perpendiculaire que possible par rapport à l'axe mécanique tibial, (qui passe par le centre des articulations), c'est-à-dire incliné par rapport au plan perpendiculaire à l'axe longitudinal du tibia tel qu'on le voit à la figure 1 et qui est lui le plan perpendiculaire à l'axe anatomique. Une fois cet angle de varus/valgus bien choisi, notamment à l'aide du capteur 10 GMCAO, le chirurgien ancre le dispositif en utilisant le deuxième alésage 2' et la deuxième vis d'ancrage. Une fois les deux vis d'ancrage ancrées dans la face latérale frontale du tibia, l'ensemble du dispositif ne peut plus tourner et est fixé en ce qui concerne l'angle de varus/valgus du plan de coupe.

Le chirurgien va maintenant, en agissant sur la molette 5, fixer l'orientation qu'il souhaite donner au plan de coupe en ce qui concerne une rotation par rapport à l'axe 12, c'est-à-dire définir la pente A/P (antéro-postérieur) qu'il souhaite donner à son plan de coupe. Pour ce faire, il agit par l'intermédiaire de la molette 5 qui va pousser ou tirer la tige 3 tranversale parallèlement à l'axe d'ancrage. La liaison de la tige 3 transversale avec les montants 7 verticaux va donc permettre de déplacer les montants 7 verticaux en rotation par rapport au corps 1. Un déplacement en translation de la tige 3 transversale se traduit par un déplacement plus important des extrémités libres des montants 7 verticaux, et donc par une rotation du plan (P) par rapport à l'axe 3. Par conséquent, le plan de coupe va plus ou moins s'incliner par rapport au corps 1 du dispositif de positionnement suivant un angle défini dans la rotation par rapport à l'axe 3. On définit ainsi ce que l'on appelle l'angle de pente A/P du plan de coupe (A/P = antéro-postérieur).

Une fois la pente A/P et l'angle de varus/valgus définis et positionnés par le chirurgien, celui-ci n'a plus qu'à définir la hauteur du plan de coupe par rapport au corps 1 fixe. Pour se faire, il agit alors sur la molette 8 qui par vissage ou dévissage de la tige 16 va faire monter ou descendre les montants 7 verticaux et donc définir la hauteur du guide de coupe (et donc du plan P de coupe) par rapport au corps 1 principal.

Toute cette description s'applique exactement de la même manière à un fémur. Un guide de coupe G de fémur est représenté à la figure 2. Comme on le voit, le guide (G') de coupe de tibia est différent du guide de coupe G car légèrement incurvé. Cependant, le dispositif de positionnement s'adapte aux deux types (et même trois types puisqu'il y a un guide de coupe gauche également pour le tibia) de guide de coupe. Plutôt que de se servir de sa propre impression, le chirurgien peut aussi utiliser le capteur 10 qui, sur un écran, retransmet d'une part la position du plan de coupe et d'autre part, à l'aide d'autres capteurs la position du plan mécanique tibial parfait. Ainsi, à l'aide d'un ordinateur, il positionne exactement sur l'écran le plan de guide de coupe par rapport à l'axe mécanique tibial parfait en les faisant se correspondre et ainsi positionne parfaitement son guide de coupe en vue d'une résection du tibia ou du fémur.

Aux figures 3 et 4 il est représenté un dispositif en variante du dispositif de la figure 1.

La différence entre les deux dispositifs est que l'une des protubérances dans lesquelles sont définis des alésages (2a aux figures 3 et 4) n'est pas montée solidaire du corps 1 principal du dispositif de positionnement, mais est montée de telle sorte qu'elle peut se déplacer en translation par rapport à celui-ci sous l'action d'une tige d'actionnement 11, elle-même actionnée par vissage par une molette 15. Ainsi, une fois que les deux vis d'ancrage ont été ancrées, on peut encore, grâce au jeu des deux ancrages, modifier légèrement la position de la vis vissée dans l'alésage 2a par rapport au corps 1 et donc légèrement modifier l'angle de varus/valgus. Evidemment, cette modification est une modification fine car elle joue sur les jeux de tolérance des ancrages. En général, il n'est pas possible de modifier l'angle de plus que plus ou moins 3°, compte tenu des jeux liés à l'ancrage des deux vis 2a, 2'. Le reste du dispositif est identique à ce qui est décrit précédemment en liaison avec les figures 1 et 2.

## Revendications

1. Dispositif de positionnement d'un plan (P) de coupe d'un guide (G ; G') de coupe d'un os, notamment d'un tibia et/ou d'un fémur, dans lequel il est prévu
des premiers moyens (2) d'ancrage, constitués d'un premier alésage (2, 2a) formé dans le corps (1) du dispositif de positionnement et d'une vis destinée à être vissée ou introduite dans le premier alésage pour être ancrée dans l'os, et destinés à ancrer le dispositif de positionnement à l'os en un premier point d'ancrage ;
des deuxièmes moyens (2') d'ancrage, constitués d'un deuxième alésage (2') parallèle au premier alésage et d'une deuxième vis destinée à passer ou à se visser dans le deuxième alésage pour pouvoir être ancrée dans l'os, et destinés à ancrer le dispositif de positionnement en un deuxième point d'ancrage sur l'os, différent du premier point d'ancrage, de manière à bloquer, en rotation le dispositif par rapport à l'axe d'ancrage défini par le premier alésage;
des premiers moyens (3, 7, 14, 5) de réglage de l'orientation dite de pente antéro-postérieure du plan de coupe en rotation par rapport à un deuxième axe (3) perpendiculaire l'axe d'ancrage ; et
des deuxièmes moyens (6, 7, 13, 16, 8) de réglage destinés à régler la hauteur du guide de coupe par rapport au dispositif le long d'un troisième axe, perpendiculaire à l'axe d'ancrage et au deuxième axe,
**caractérisé en ce que** les premiers moyens de réglage sont constitués de deux montants (7), montés sensiblement perpendiculairement à l'axe d'ancrage et reliés au guide de coupe, d'un montant (3) transversal reliant les deux montants l'un à l'autre, et d'une tige (14) agissant sur le montant (3) transversal pour le déplacer par rapport au corps du dispositif de positionnement dans la direction de l'axe d'ancrage, cette translation se transformant en une rotation du plan de coupe dans un petit domaine de rotation, ce qui permet de bien régler le plan de coupe A/P (antéro-postérieur)

2. Dispositif de positionnement suivant la revendication 1, **caractérisé en ce que** les deux montants (7) verticaux coulissent par rapport au corps principal dans la troisième direction sous l'action d'une molette (8), ce qui permet de régler la hauteur du guide de coupe auquel ils sont fixés par l'intermédiaire des moyens de fixation par rapport au corps du guide de coupe et donc par rapport à l'os auquel le corps de guide de coupe est ancré.

3. Dispositif de positionnement suivant la revendication 1 ou 2, **caractérisé en ce qu'**il est prévu des moyens fins de l'angle de rotation par rapport à l'axe d'ancrage.

4. Dispositif de positionnement suivant la revendication 3, **caractérisé en ce que** ces moyens de réglage fins sont constitués par le fait que le premier alésage (2a) est monté mobile sous l'action d'une tige (11) actionnée par une molette (15) par rapport au corps (1) principal du dispositif.

5. Dispositif de positionnement suivant la revendication 1, **caractérisé en ce que** le guide de coupe ou le dispositif de positionnement du guide de coupe comporte un capteur (10) permettant de positionner dans l'espace le plan (P) de coupe, notamment un système dit GMCAO.

6. Dispositif de positionnement suivant l'une des revendications 1 à 5, **caractérisé en ce qu'**il comporte des moyens de fixation, notamment amovibles, au guide de coupe constitués notamment d'ergots (7a) d'encliquetage s'encliquetant dans des logements (7b) respectifs dans les guides de coupe.

7. Dispositif suivant l'une des revendications précédentes, **caractérisé en ce que** le dispositif positionne le guide de coupe pour un fémur et pour un tibia.

8. Dispositif suivant l'une des revendications précédentes, **caractérisé en ce que** la tige (14) est en partie filetée et commandée par une molette (5).

## Claims

1. Device for positioning a cutting plane (P) of a bone cutting guide (G; G'), notably of a tibia and/or of a femur, in which provision is made for
first means of anchoring (2), consisting of a first hole (2, 2a) formed in the body (1) of the positioning device and a screw intended to be screwed or inserted into the first hole to be anchored in the bone, and intended to anchor the positioning device to the bone into an initial anchoring point;
second means (2') of anchoring, consisting of a second hole (2') parallel to the first hole and of a second screw intended to pass or to be screwed into the second hole to be able to be anchored in the bone, and intended to anchor the positioning device into a second anchoring point on the bone, different to the first anchoring point, so as to block in rotation the device with respect to the anchoring axis defined by the first hole;
first means (3, 7, 14, 5) of adjustment of the so-called orientation of the antero-posterior slope of the cutting plane in rotation with respect to a second axis (3) perpendicular to the anchoring axis; and
second means (6, 7, 13, 16, 8) of adjustment intended to adjust the height of the cutting guide with respect to the device along a third axis, perpendicular to the anchoring axis and the second axis,
**characterised in that** the first means of adjustment consist of two uprights (7), mounted largely perpendicularly to the anchoring axis and connected to the cutting guide, of a transverse upright (3) connecting the two uprights to one another, and a rod (14) acting on the transverse upright (3) to move it with respect to the body of the positioning device in the direction of the anchoring axis, with this translation being transformed into a rotation of the cutting plane in a small field of rotation, which permits the A/P (antero-posterior) cutting plane to be properly adjusted.

2. Positioning device in accordance with claim 1, **characterised in that** the two vertical uprights (7) slide with respect to the main body in the third direction under the action of a adjustment knob (8), which permits the height of the cutting guide to which they are fixed to be adjusted through the medium of means of fixing with respect to the body of the cutting guide and hence with respect to the bone to which the cutting guide body is anchored.

3. Positioning device in accordance with claim 1 or 2, **characterised in that** provision is made for fine means of the angle of rotation with respect to the anchoring axis.

4. Positioning device in accordance with claim 3, **characterised in that** these fine means of adjustment consist of the fact that the first hole (2a) is mounted mobilely under the action of a rod (11) activated by a adjustment knob (15) with respect to the main body (1) of the device.

5. Positioning device in accordance with claim 1, **characterised in that** the cutting guide or the positioning device of the cutting guide includes a sensor (10) permitting the positioning in space of the cutting plane (P), notably a system called GMCAO.

6. Positioning device in accordance with one of claims 1 to 5, **characterised in that** it comprises notably moveable means of fixing to the cutting guide, consisting notably of locking pins (7a) locking into the respective placements (7b) in the cutting guide.

7. Device in accordance with one of the previous claims, **characterised in that** the device positions the cutting guide for a femur and for a tibia.

8. Device in accordance with one of the preceding claims, **characterised in that** the rod (14) is in part threaded and controlled by a adjustment knob (5).

## Patentansprüche

1. Vorrichtung zum Positionieren der Schnittebene (P) einer Schnittführung (G; G') für einen Knochen, insbesondere für eine Tibia und/oder einen Femur, bei welcher vorgesehen sind:
- erste Verankerungsmittel (2), die aus einer im Körper (1) der Positionierungsvorrichtung ausgebildeten ersten Bohrung (2, 2a) und aus einer Schraube bestehen, die dazu bestimmt ist, in die erste Bohrung eingeschraubt bzw. eingeführt zu werden, um in dem Knochen verankert zu werden, und die dazu bestimmt sind, die Positionierungsvorrichtung am Knochen in einem ersten Verankerungspunkt zu verankern,
- zweite Verankerungsmittel (2'), die aus einer parallel zur ersten Bohrung verlaufenden zweiten Bohrung (2') und einer zweiten Schraube bestehen, die dazu bestimmt ist, in die zweite Bohrung eingeführt bzw. eingeschraubt zu werden, um in den Knochen verankert zu werden, und die dazu bestimmt sind, die Positionierungsvorrichtung am Knochen in einem zweiten Verankerungspunkt zu verankern, der von dem ersten Verankerungspunkt verschieden ist, so dass die Vorrichtung bezüglich der von der ersten Bohrung definierten Verankerungsachse drehfest gesichert ist;
- erste Einstellmittel (3, 7, 14, 5) zum Ausrichten der sogenannten antero-posterioren Neigung der Schnittebene bezüglich einer Drehung um eine zweite Achse (3), die senkrecht zur Verankerungsachse verläuft, und
- zweite Einstellmittel (6, 7, 13, 16, 8), die dazu bestimmt sind, die Höhe der Schnittführung bezüglich der Vorrichtung entlang einer dritten Achse einzustellen, die senkrecht zur Verankerungsachse und zur zweiten Achse verläuft,
**dadurch gekennzeichnet, dass** die ersten Einstellmittel aus zwei Stützen (7), die im wesentlichen senkrecht zur Verankerungsachse montiert und mit der Schnittführung verbunden sind, aus einer Querstrebe (3), welche die beiden Stützen miteinander verbindet, und aus einem Schaft (14) bestehen, der auf die Querstrebe (3) einwirkt, um diese bezüglich des Körpers der Positionierungsvorrichtung in Richtung der Verankerungsachse zu verschieben, wobei diese Verschiebung in eine Drehung der Schnittebene in einem kleinen Drehbereich umgewandelt wird, wodurch es möglich ist, die Schnittebene A/P (antero-posterior) richtig einzustellen.

2. Vorrichtung zum Positionieren nach Anspruch 1, **dadurch gekennzeichnet, dass** die beiden senkrechten Stützen (7) unter der Wirkung eines Rändelrads (8) bezüglich des Hauptkörpers in der dritten Richtung gleitbeweglich sind, wodurch es möglich ist, die Höhe der Schnittführung einzustellen, an der sie über Befestigungsmittel bezüglich des Körpers der Schnittführung und somit bezüglich des Knochens befestigt sind, an welchem der Körper der Schnittführung verankert ist.

3. Vorrichtung zum Positionieren nach Anspruch 1 oder 2, **dadurch gekennzeichnet, dass** Feineinstellmittel für den Drehwinkel bezüglich der Verankerungsachse vorgesehen sind.

4. Vorrichtung zum Positionieren nach Anspruch 3, **dadurch gekennzeichnet, dass** diese Feineinstellmittel darin bestehen, dass die erste Bohrung (2a) unter der Wirkung eines Schafts (11) beweglich gelagert ist, der über ein Rändelrad (15) bezüglich des Hauptkörpers (1) der Vorrichtung betätigt wird.

5. Vorrichtung zum Positionieren nach Anspruch 1, **dadurch gekennzeichnet, dass** die Schnittführung bzw. die Positionierungsvorrichtung für die Schnittführung einen Sensor (10) enthält, mit dem die Schnittebene (P) räumlich positioniert werden kann, insbesondere ein sogenanntes GMCAO-System (Geste Medico-Chirurgicaux Assistés par Ordinateur - Computer Assisted Surgery).

6. Vorrichtung zum Positionieren nach einem der Ansprüche 1 bis 5, **dadurch gekennzeichnet, dass** sie insbesondere abnehmbare Befestigungsmittel zur Befestigung an der Schnittführung enthält, die insbesondere aus Rastvorsprüngen (7a) bestehen, die in jeweilige Aufnahmen (7b) in den Schnittführungen einrasten.

7. Vorrichtung zum Positionieren nach einem der vorangehenden Ansprüche, **dadurch gekennzeichnet, dass** die Vorrichtung die Schnittführung für einen Femur und für eine Tibia positioniert.

8. Vorrichtung zum Positionieren nach einem der vorangehenden Ansprüche, **dadurch gekennzeichnet, dass** der Schaft (14) aus einem Gewindeteil besteht und über ein Rändelrad (5) betätigt wird.
